(19) Europäisches Patentamt
European Patent Office
Office européen des brevets

(11) Publication number: **0 159 628**
**B1**

(12) # EUROPEAN PATENT SPECIFICATION

(45) Date of publication of patent specification: **18.07.90**

(51) Int. Cl.$^5$: **A 61 K 7/11**

(21) Application number: **85104416.4**

(22) Date of filing: **11.04.85**

(54) **Hair stengthening and permanent waving composition.**

(30) Priority: **12.04.84 US 599296**

(43) Date of publication of application:
**30.10.85 Bulletin 85/44**

(45) Publication of the grant of the patent:
**18.07.90 Bulletin 90/29**

(84) Designated Contracting States:
**AT BE CH DE FR GB IT LI LU NL SE**

(56) References cited:
**WO-A-82/02337**
**AT-B- 285 054**
**DE-A-2 542 338**
**GB-A-1 298 237**
**US-A-3 208 910**
**US-A-4 344 763**

**The file contains technical information submitted after the application was filed and not included in this specification**

(73) Proprietor: **Revlon, Inc.**
**767 Fifth Avenue**
**New York, N.Y.10022 (US)**

(72) Inventor: **Stadnick, Richard P.**
**108 Derfuss Lane**
**Blauvelt New York (US)**

(74) Representative: **Körber, Wolfhart, Dr.rer.nat. et al**
**Patentanwälte Dipl.-Ing. H. Mitscherlich Dipl.-Ing. K. Gunschmann Dr.rer.nat. W. Körber Dipl.Ing. J. Schmidt-Evers Dipl.-Ing. W. Melzer**
**Steinsdorfstrasse 10**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to hair strengthening and permanent waving compositions, and more particularly, to reactive silicone hair strengthening and waving compositions.

Hair care is one of the most important parts of beauty care, and a large variety of hair treatment products are used for providing such care in the form of pre-shampoo conditioners, shampoos, after-shampoo conditioners, rinses, setting lotions, sprays, dyes, bleaches, permanent wave agents and the like. These products in addition to providing the desired result in the hair, such as luster, curl combability, softness, color and an overall appealing look, also do damage to the hair, especially when used indiscriminantly without professional guidance. Damage to hair may also result from other sources, such as combing, humidity, dryness, dirt, sunrays, such as u.v. and infrared radiation, and pollution in the atmosphere. However, damage to hair occurs mostly in the form of physical and chemical changes in hair as a result of bleaching, oxidative dying, hair relaxing via alkaline relaxers and reducing waving and curling preparations.

Hair damage may occur slowly and in various ways with frequent "treatment" of the hair. Over an extended time period the use of various chemicals in the hair treatment products will effect a weakened hair structure. Illustrative of this undesirable effect is that which occurs when the natural configuration of hair is altered from curled to straight or vice versa. The hair is subjected to the action of a composition which relaxes the hair by rupturing the disulfide bonds in the keratin of the hair to produce free sulfhydryl groups. This breakage in the disulfide diminishes the rigidity of the hair proteins, leaving the hair pliable and soft. The compositions most commonly used as relaxers are based on sodium hydroxide, sulfite, or thioglycolate. When a thioglycolate based composition is used as the relaxer, the hair is treated with an oxidant, such as peroxide, to impart rigidity to the hair. These operations, particularly the reduction step, can be carried out at either room or elevated temperature. Both types of treatment, the cold as well as the hot, require visual evaluation of the hair. Ultimately, the quality of the hair, straightened or curled, tests on the subjective judgement of the salon operator, and more often than not because of the subjective element, the hair is either underprocessed or overprocessed.

In addition to the problem of being underprocessed or overprocessed, the hair subjected to oxidative hair preparations, lacks the desired texture, it is raspy, troublesome to comb, and require further conditioning. To correct these conditions some manufacturers of hair treatment products frequently supply post-treatment conditioners usually as part of a kit included with the acidic oxidative preparation. Other incorporate specific hair conditioning agents compatible with the oxidizing agents, such as non-ionic and cationic agents.

Hair conditioning agents are used not only in conjunction or after treatment with oxidizing agents but also to generally assist in the control and management of hair as for example after shampooing. Conditioned hair is easily untangled and combed through after shampooing, lays orderly when dry and provides a favorable feeling to the touch. The conditioning action on hair, particularly by cationic conditioning agents, is believed to be caused by the attraction of the positively charged agent to the negative sites on hair protein resulting in the deposition of the agent on to the hair fiber.

Cationic agents or surfactants have been used extensively as hair conditioning agents in creme rinses and shampoos. Best results have been obtained with cationic surfactants that are long chain high molecular weight quaternary compounds or long chain fatty amine salts. The positive charge of the quaternary surfactant is attracted to the negatively charged surface of the hair protein giving it lubricity during wet combing and a desirable texture after drying.

Other types of agents for deposition on hair used in the prior art are the organo silicones. The organo silicones, having hydrolyzable groups on the silicon atom, hydrolyze to the corresponding silanol and condense to an organo-silicone polymer on the hair fibers coating the same and providing body thereto are used for both conditioning and setting the hair.

It has also been proposed to use epoxy-alkyl-siloxanes according to GB—A—990,496.

GB—A—1,298,237 describes alcoholic and aqueous alcoholic solutions of epoxy-organo-silane hair waving agents of general formula

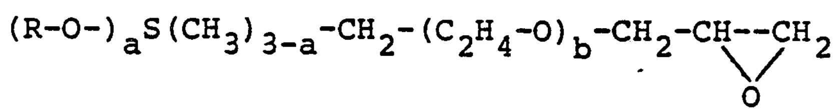

in which

a is 2 or 3;

b is 0 or 1; and

R is an alkyl radical with 1 to 6 carbon atoms or an ether radical of the formula

$$-(C_2H_4-O)_n-CH_3$$

in which n is 1, 2 or 3.

The hair waving agents described in the GB—A—1,298,237 undergo hydrolysis to the corresponding alcohols and the alcohols then undergo condensation to form organosiloxanes, the epoxide group meanwhile reacting with the hair. Said epoxy-organo-silane reacts with hair. That means that the chemical mechanism of this reaction mainly involves the epoxide group.

While the above mentioned conditioning agents provide substantial benefits to hair, none is directed to strengthening the hair structure and thereby restore the hair, as much as possible, to its natural strength.

The foregoing discussion illustrates some of the problems associated with hair care and solutions provided therefor by the prior art.

The present invention addresses the problem of treating the weakened hair, whether such condition is caused by nature, the environment or hair treatment preparations applied thereto.

The present invention relates to a composition for strengthening the tensile strength of hair comprising

a, an alkyltrialkoxysilane having the formula

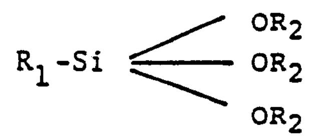

wherein

$R_1$ is an aliphatic radical of $C_1$—$C_{18}$ carbon atoms, such as methyl, ethyl, propyl, butyl, hexyl, octyl, decyl, octadecyl; a substitute aliphatic radical selected from 3-aminopropyl, N,N-diethyl-3-aminopropyl, and N-(propyl) urea; or an alkenyl radical, such as vinyl or allyl;

$R_2$ is an aliphatic radical of $C_1$—$C_4$ carbon atoms, such as methyl, ethyl, propyl and butyl;

b, a solvent of said alkyltrialkoxysilane. The solvent may consist of water, ethanol, isopropyl alcohol, nonoxynol-4, or an aqueous solution of ethanol, isopropyl alcohol or nonoxynol-4.

To obtain a composition according to the present invention, a solution is prepared by adding an alkyltrialkoxysilane to the appropriate solvent with mixing. The composition preferably comprises 0.5 to 40% v/v and more preferably 2 to 10% v/v of an alkyltrialkoxysilane in the solvent. The preferred solvent is an aqueous solution of ethanol, isopropyl alcohol or nonoxynol-4 in which the water constitutes no more than 20% v/v of the total solvent volume.

Although the silanes described herein are stable for several days in alcohol/water phases having from 0—20% water, it is preferred to prepare the solution just prior to application to the hair. If the solution is allowed to stand in the presence of water for prolonged time periods, excessive polymerization and premature precipitation of the silane may occur, thus rendering the composition less effective for its intended use.

The method of application of the composition includes applying the composition to the hair by submerging the clean hair in the composition or applying the composition with an applicator and working it into the hair with the fingers and a comb. The process is completed by blow drying the hair with hot air.

The present invention is based on the discovery that the reactive alkyltrialkoxysilane containing three labile alkoxy groups in the presence of water hydrolyzes to form three reactive silanol groups which in turn condense with each other to form oligomers. The oligomers then hydrogen bond with functional groups containing active hydrogens (hydroxyl, amino, carboxyl, sulfhydryl and the like) in the hair. During drying or curing a stable covalent bond is formed with the hair with concomitant loss of water. While the curing process will occur at room temperature, it is preferred to speed up the reaction by drying the hair with a hot air dryer. The application of heat speeds up evaporation of the solvent and the by-products of the reaction. Once water has been removed from the hair, the resulting bonds become stable to hydrolytic action. The degree and rate of polymerization of the silane is determine by the amount of water available and the organic substituent of the silane.

This characteristic of the silanes of the present invention allows for their application to hair for increasing hair strength, retaining hair configuration such as curls, and providing manageability to hair.

The silanes of the present invention are either commercially available or can be prepared by methods known in the art using commercially available starting materials and reagents.

Silanes are readily solubilized in ethanol, isopropyl alcohol and many other organic solvents, however, for the present invention the preferred solvent for the silanes is an aqueous/alcohol mixture containing up to 20% by volume of water. This composition ensures that a sufficient amount of water is available to hydrolyze the silane and possibly swell the hair fibers for increased penetrations of the active ingredient prior to the polymerization step. Silanes show unexpected stability to water in such vehicles as determined by the time to hazing (precipitation of siloxane polymers). For example, solutions of ethyltriethoxysilane dissolved in 80/20 ethanol/water remain clear for several days. As the percentage by volume of water increases beyond 20% of the total solvent composition, more rapid hazing and precipitation of the siloxane polymer is observed to occur, thus rendering the composition less effective for its intended use.

Test methods used in connection with the invention and working examples follow.

Tensile Stength

This test relates to the determination of the force required to break (tensile strength at breakpoint) the hair sample by using a constant elongation rate tester such as the Instron Tensile Tester. The test is performed as follows:

Each tress of hair to be tested is conditioned in a 50% relative humidity chamber for at least 24 hrs prior to use. Twenty strands of hair, randomly selected from each tress, are used for the test. A 5,08 × $10^{-2}$m (two-inch) long section is cut from each hair strand and weighed to the nearest 0.1 microgram. The weighed cut sections are placed into distilled water and allowed to hydrate for at least 16 hours. Each hydrated hair sample is placed between the jaws of the Instron Tensile Tester, which are preset at 2,54 × $10^{-2}$m (1 inch) apart, and clamped securely. The tensile strength is then determined according to the manufacturer's instructions.

The tensile strength is the force required to break the hair strand. This force is expressed in terms of grams force per cross sectional area of hair strand.

Method For Determination of Silane Content in Hair

The silicon content of treated hair is determined colorimetrically as silicomolybdate or as molybdenum blue and the result calculated as silane. Approximately 0.7 gram ± 0.1 mg of hair is weighed accurately into a breaker, to which 5 ml of concentrated nitric acid is added. The sample is heated to dryness and the addition of nitric acid and heating steps are repeated until a white residue remains in the beaker. Then 10 ml of 1:10 hydrochloric acid is added to the beaker and the content is filtered with suction through a membrane filter. The beaker is thoroughly washed with 1:10 hydrochloric acid and the liquid is filtered. The filter is placed in the bottom of a polyethylene beaker and 1.0 ml of hydrofluoric acid is added to cover the filter. The beaker is then covered and allowed to stand for 30 min. Then 25 ml of water and 50 ml of boric acid solution is added with stirring, and the solution is heated at 40°C in a water bath for at least 10 min. Four (4) ml of molybdic acid reagent is added while stirring. Then 20 min. later, 20 ml of 10N sulfuric acid is added with stirring. If any yellow color persists, the solution is read within 2 min. after acidification in a spectrophotometer at 420 nm against distilled water. If a colorless solution results, the solution is allowed to stand for 2 to 5 min. and 1 ml of 1-amino-2-naphththol-4-sulfonic acid reagent is added, which is then mixed and read after 20 min. at 820 nm.

A calibration curve in the yellow silicomolybdate concentration range (420 nm) is made by diluting 0.5, 1, 2 and 3 ml aliquots of the stock silicon (1 mg/ml) standard to 25 ml in polyethylene beakers and proceeding as hereinbefore described from and including the step of heating the solution for at least 10 min. After completing the steps as indicated, absorbence is read at 420 nm. Absorbance against mg silicon is then plotted. A calibration curve is also prepared in the molybdenum blue concentration range (820 nm) by diluting 50, 100, 200 and 500 microliters of the silicon stock standard (1 mg/ml) to 25 ml in polyethylene beakers and proceeding as described above for the silicomolybdate calibration curve. Absorbance is read at 820 nm.

The concentration of silicon is obtained from the appropriate calibration curve. The calculation is as follows:

$$\text{Percent silane} = \frac{\text{mg silicon}}{\text{grams of hair}} \times \frac{\text{mole weight of silane}}{\text{atomic weight of silicon}} \times 0.1$$

Example 1

A hair sample previously weakened by sodium hydroxide was submerged in 3.0% v/v solution of ethyltriethoxysilane (obtained from Petrarch Systems, Inc., Levittown, PA) in a mixture of 80/20 ethanol/water. The hair was rinsed free of excess silane and cured by blow drying with a hot air dryer for about ten minutes. The hair was tested using the above-described test procedures. The results were as follows:

a. The hair treated with sodium hydroxide solution showed 11 to 14% decrease in tensile strength;

b. The sodium hydroxide weakened hair which subsequently was treated with the silane-containing composition increased in tensile strength back to within 97 to 98% of its original (untreated) tensile strength;

c. The silane treated sodium hydroxide weakened hair picked up between 0.4 to 1.4% silicon by weight, calculated as ethyltriethoxysilane.

Example 2

A 95% v/v solution of ethyltriethoxysilane in nonoxynol-4 was prepared. Immediately prior to treating the hair, 40 parts of the silane/nonoxynol-4 phase was added with stirring to 60 parts of water. The hair, which was previously weakened by sodium hydroxide was completely wetted with the silane/nonoxynol-4/water solution. The solution was allowed to remain on the hair for 30 minutes. The hair was rinsed free of excess solution and blow dried with a hot air drier.

Upon testing, the data showed a 10% increase in tensile strength of the hair.

Example 3

A swath of virgin hair was heavily damaged by a 45 minute treatment with a 2% aqueous solution of

sodium hydroxide. The alkali-damaged swatch was washed with a pH 7.0 shampoo to restore the acid balance, allowed to dry under ambient conditions and subdivided into a series of equal-sized tresses. Three tresses were selected and each treated for 30 minutes with 10 grams of the silane-containing composition of Example 2, cured and blow dried. The procedure used to treat the three tresses was exactly the same except for the manner in which the excess silane was removed. Instron analyses were carried out on all of the tresses. The result and the manner of removing excess silane are shown in Table I.

Table I
Instron Results for Prepared Tresses

| Sample | n | Tensile Strength (G) X* | SEM** |
|---|---|---|---|
| Control virgin hair | 20 | 29.49 | ± 0.61 |
| Control alkali-damaged hair | 20 | 14.48 | ± 1.07 |
| Alkali-damaged hair, silane treated, rinsed, then cured | 20 | 17.51 | ± 0.56 |
| Alkali-damaged hair, silane treated, cured, then rinsed | 20 | 16.56 | ± 0.59 |
| Alkali-damaged hair silane treated, cured, then rinsed and shampooed | 20 | 16.95 | ± 0.702 |

n denotes the number of determinations here and in subsequent tables.
X* denotes the mean of the number of measurements here and in subsequent tables.
SEM** denotes the standard error of the mean here and in subsequent tables.

The data in Table I shows that all the silane treated tresses have significantly increased in strength (about 15—20%) relative to the alkali-relaxed control. No significant difference in hair strength is shown among the silane-treated tresses indicating that the mode by which excess reagent is removed from the hair has no effect on hair strength.

It was also discovered that if hair, arranged in a desired configuration with curling rollers or rods, is treated with a silane-containing composition of the present invention and blow dried, the silane bonding to the hair not only increases hair strength but also maintains the hair in the shape in which it was dried. This effect is shown for both alkali-weakened and virgin hair. The resulting permanent wave resists the tendency to revert to the initial configuration even after repeated shampooing.

Example 4

A dark brown virgin hair swatch obtained from a single lot was washed with a 10% sodium lauryl sulfate solution, rinsed with tap water, and blow dried at room temperature. The swatch was subdivided into eighteen tresses ten inches long each weighing one gram. One tress was selected to serve as virgin control and five others were set aside for silane treatment. The remaining twelve tresses were set aside for the alkali treatment. Before treatment, each tress was gently combed and separately rolled on curling rods. Alkali treatment was carried out by submerging the rolled tresses in a 2.0% sodium hydroxide solution for one hour. Subsequently, each tress was rinsed under running tap water for at least 10 minutes. The rolled tresses were towel dried, soaked in pH 7.0 buffer for one hour to restore the acid balance, and rinsed out thoroughly to remove the buffer. The rolled tresses were finally dried at room temperature.

Equimolar solutions of silanes denoted in Tables II and III were prepared separately in a mixture of 80:20 ethanol/$H_2O$, to carry out silylation. The virgin and alkali treated tresses on rollers were treated separately for 30 minutes with 100 ml of 0.26 M solution of silane. The rolled tresses were blow dried for four minutes after the silane treatment with a 1600-watt blow drier and thoroughly sprayed with water. The rolled tresses were allowed to stand for five minutes and finally blow dried for 15 minutes at the high temperature setting. Unsilylated virgin and alkali control were also subjected to the same treatment. The

hair tresses, after equilibrating to room temperature, were carefully unwound from the curling rods and mounted on the curl retention board. The mounted specimens were then incubated at 37,8°C (100°F)/95% relative humidity. Curl retention data were obtained periodically over 24 hours. The tresses were removed from the board, wetted under running tap water for 1 minute, dried and soaked in a beaker of water for 30 additional minutes, removed, air dried, twice shampooed and rinsed again. The final curl length and patterns of each tress were evaluated to determine curl stability. Data and comments are summarized in Tables II and III.

Table II
Alkali Pre-Treated Hair

| Sample | Silane[(1)] Conc. (%) | n | % Curl Retention (X) | SEM | Comments |
|---|---|---|---|---|---|
| Control (alkali relaxed) | 0.00 | 2 | 12.27 | ±1.35 | Good curl and S-wave pattern No shine |
| Octadecyl triethoxysilane | 10.84 | 2 | 14.55 | ±0.13 | Very good S-wave pattern Good shine |
| 3-Aminopropyl triethoxysilane | 4.70 | 2 | 17.80 | ±0.20 | Very good S-wave pattern Good shine |
| N-(triethoxy silylpropyl)urea | 6.90 | 2 | 16.36 | ±0.42 | Very good curl and S-wave pattern |

(1) All solutions contain equal moles of silanes (0.26M)

Table - III

Virgin Hair

| Sample | Silane[1] Conc. (%) | n | % Curl Retention (x) | Comments |
|---|---|---|---|---|
| Control virgin hair | 0.00 | 1 | 7.16 | Almost no curl, no S-wave pattern |
| Octadecyltriethoxy-silane | 10.84 | 1 | 12.73 | Very good body wave |
| Octyltriethoxy-silane | 7.20 | 1 | 11.04 | Fair body wave |
| 3-Aminopropyltri-ethoxysilane | 4.70 | 1 | 11.35 | Good body wave |
| Vinyltriethoxy-silane | 4.90 | 1 | 11.94 | Good body wave |
| N-(triethoxy-silylpropyl)urea | 6.90 | 1 | 10.38 | Fair body wave |

(1) All solutions contain equal moles of silanes (0.26M)

Example 5

Series of tresses were prepared from a single lot of dark brown virgin hair, each tress being 1.5 ± 0.1 g in weight and 0,254 m (10") long. Two of the tresses, selected at random to serve as permanent waved controls, were treated with Collagen Amino Acid Professional Formula® (active ingredients: 9.2% ammonium thioglycolate, 10% sodium bromate). Fourteen virgin hair tresses were wrapped on rods and immersed for 1 hour in a 2% sodium hydroxide solution. The wrapped tresses, after having been rinsed free of alkali, were treated with pH 7.0 buffer to restore the acid balance to the hair. The tresses were rinsed free of buffer and treated as described in Tables IV and V. The procedure used for the evaluation and curing of alkali-treated hair was described in Example 4. Heat was also applied to the control samples for an identical period of time. All the prepared tresses were allowed to equilibrate under ambient conditions of temperature and humidity.

After equilibration, the tresses were carefully unwound from their curling rods and their respective curl lengths carefully measured to the nearest 0.1 cm. The tresses, after having been mounted on a curl retention panel, were incubated for 24 hours at 37,8°C (100°F)/95% RH. Next, the tresses were removed from the panel, flushed with running tap water for 1 minute, soaked in a beaker of water for 30 additional minutes, removed and shampooed twice with Flex Shampoo®, rinsed free of soap and allowed to air dry. The final curl length and pattern of each tress were carefully evaluated to determine curl stability.

Results are shown in Tables IV and V.

Table - IV

Curl Retention

| Sample | n | % Curl Retention (X) | SEM | Comments |
|---|---|---|---|---|
| Alkali relaxed control | 1 | 10.2 | - | Little curl - no S-wave pattern |
| Alkali relaxed/5% ETES[1] | 1 | 15.4 | - | Good S-wave pattern |
| Alkali relaxed/30% ETES[1] | 1 | 15.0 | - | Good S-wave pattern |
| Alkali relaxed/urea swelled-30% ETES[1] | 1 | 13.4 | - | Good S-wave pattern |
| Alkali relaxed/salt swelled-30% ETES[1] | 1 | 14.2 | - | Good S-wave pattern |
| Alkali relaxed/30% ETES[1] in DET[2] | 2 | 15.2 | ±0.20 | Very good S-wave pattern |
| Alkali relaxed/30% ETES[1] in DMF[3] | 2 | 14.1 | ±0.20 | Very tight curl |
| Permanent waved controls | 2 | 16.7 | ±0.60 | Very good S-wave pattern |

(1) ETES = Ethyltriethoxysilane in 80/20 alcohol/water in here and in Table V

(2) DET = Diethyltoluamide

(3) DMF = Dimethylformamide

Table - V

| Sample | n | % Curl Retention (x) | SEM | Comments |
|---|---|---|---|---|
| Virgin hair control | 2 | 6.9 | ±0.60 | Very little curl, no S-wave pattern |
| Virgin hair/5% ETES | 2 | 12.2 | ±0.80 | Good body wave |
| Virgin hair/salt swelled 5% ETES[1] | 2 | 12.6 | ±0 | Good body wave |
| Virgin hair/urea swelled 5% ETES[2] | 2 | 12.6 | ±0 | Good body wave |
| Alkali relaxed control | 2 | 7.5 | ±0 | Very little curl, no S-wave pattern |
| Alkali relaxed/5% ETES | 2 | 12.2 | ±0 | Good S-wave pattern |
| Alkali relaxed/urea swelled 5% ETES | 2 | 11.4 | ±0 | Good S-wave pattern |
| Alkali relaxed/salt swelled 5% ETES | 2 | 10.4 | ±1.00 | Fair S-wave pattern |

(1) Hair was swelled with a 2.8M LiCl/0.5M $Na_2SO_4$ aqueous solution prior to treating the hair with silane.

(2) Hair was swelled with a 4M aqueous solution of urea prior to treating the hair with silane.

Table IV demonstrates that silylation of alkali treated hair on curling rods produces a curl pattern equivalent to that produced by a conventional permanent waving treatment, and is substantially greater than the curl produced in the alkaline relaxed control. Curl "memory" was retained in the alkali-silylated tresses even after repeated shampooing.

Table V demonstrates that silane compositions of the present invention impart a good stable curl to alkali treated hair and, in addition, that the curl retention of the virgin hair is significantly improved after silylation through the formation of a stable body wave pattern.

**Claims**

1. A composition for strengthening the tensile strength of hair comprising:

a., an alkyltrialkoxysilane having the formula

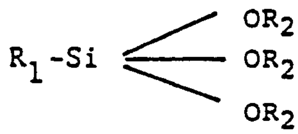

wherein

$R_1$ is an aliphatic radical of $C_1$—$C_{18}$ carbon atoms; a substituted aliphatic radical selected from 3-aminopropyl, N,N-diethyl-3-aminopropyl, N-(propyl)urea or an alkenyl;

$R_2$ is an aliphatic radical of $C_1$—$C_4$ carbon atoms; and

b., a solvent of said alkyltrialkoxysilane.

2. The composition of claim 1 wherein the aliphatic radical is methyl, ethyl, propyl, butyl, hexyl, octyl, decyl or octadecyl.

3. The composition of claim 1 wherein the alkenyl is vinyl or allyl.

4. The composition of any of claims 1 to 3 wherein the solvent is water, ethanol, isopropyl alcohol or nonoxynol-4.

5. The composition of any of claims 1 to 3 wherein the solvent is an aqueous solution containing ethanol, isopropyl alcohol or nonoxynol-4.

6. The composition of claim 5 wherein water constitutes no more than about 20% v/v of the solvent.

7. The composition of any of claims 1 to 6 wherein the alkyltrialkoxysilane is present in about 0.5 to 40% v/v based on the total volume of the composition.

8. The composition of any of claims 1 to 7 wherein based on the total volume of the composition alkyltrialkoxysilane is present in an amount from about 2 to 10% v/v in 98—90% v/v of a solvent.

9. The composition of any of claims 1 to 8 wherein the alkyltrialkoxysilane is octadecyltriethoxysilane, n-octyltriethoxysilane, 3-aminopropyltriethoxysilane, vinyltriethoxysilane, n-(triethoxysilylpropyl) urea or ethyltriethoxysilane.

10. A method of strengthening hair comprising the steps of:

a., applying to the hair an effective amount of the composition of any claims 1 to 9

b., applying heat to the hair to effect curing.

11. The method of claim 10 wherein the hair is an alkali-weakened hair.

12. The method of claim 10 or 11 wherein the hair is virgin hair.

13. The method of any of claims 10 to 12 wherein prior to step (a) the hair is set to the desired configuration.

14. The method of any of claims 10 to 13 wherein the hair is pre-treated with an alkali or a thioglycolate prior to applying the composition.

**Patentansprüche**

1. Eine Verbindung zur Stärkung der Reißfestigkeit der Haare, die beinhaltet

(a) eine Alkyltrialkoxysilanverbindung mit der Formel

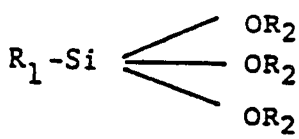

dadurch gekennzeichnet, daß

$R_1$ ein aliphatischer Rest mit 1—18 Kohlenstoffatomen, ein substituierter aliphatischer Rest ausgewählt aus 3-Aminopropyl, N,N-Diethyl-3-aminoprospyl, N-(Propyl)harnstoff oder ein Alkenylrest ist,

$R_2$ ein aliphatischer Rest mit 1—4 Kohlenstoffatomen ist; und

(b) ein Lösungsmittel für die besagten Alkyltrialkoxysilanverbindungen ist.

2. Die Zusammensetzung nach Anspruch 1, dadurch gekennzeichnet, daß der aliphatische Rest ein Methyl, Ethyl, Propyl, Butyl, Hexyl, Octyl, Decyl oder Octadecyl ist.

3. Die Verbindung nach Anspruch 1, dadurch gekennzeichnet, daß das Alkenyl ein Vinyl oder Allyl ist.

4. Die Zusammensetzung nach allen Ansprüchen 1 bis 3, dadurch gekennzeichnet, daß das Lösungsmittel Wasser, Ethanol, Isopropanol oder Nonoxyl-4 ist.

5. Die Zusammensetzung nach allen Ansprüchen 1 bis 3, dadurch gekennzeichnet, daß das Lösungsmittel aus einer wäßrigen Lösung mit Ethanol, Isopropanol oder Nonoxylnol-4 besteht.

6. Die Zusammensetzung nach Anspruch 5, dadurch gekennzeichnet, daß der Wassergehalt nicht mehr als 20% v/v des Lösungsmittelvolumens betragen darf.

7. Die Zusammensetzung nach allen Ansprüchen 1 bis 6, dadurch gekennzeichnet, daß der Anteil der Alkyltrialkoxysilanverbindung 0.5 bis 40% v/v der totalen Zusammensetzung beträgt.

8. Die Zusammensetzung nach allen Ansprüchen 1 bis 7, dadurch gekennzeichnet, daß basierend auf einem Endvolumen die Alkyltrialkoxysilanverbindung in einer Menge von 2 bis 10% v/v in 98—90% v/v Lösungsmittel vorliegt.

9. Die Zusammensetzung nach allen Ansprüchen 1 bis 8, dadurch gekennzeichnet, daß die Alkyltrialkoxysilanverbingungen gleich Octadecyltriethoxysilan, n-Octyltriethoxysilan, 3-Aminopropyltriethoxysilan, Vinyltriethoxysilan, n-(Triethoxysilylpropyl)harnstoff oder Ethyltriethoxysilan ist.

10. Verfahren zur Stärkung der Haare, die folgende Schritte beinhaltet:

(a) Auftragen einer effektiven Menge der Zusammensetzung aller Ansprüche nach 1 bis 9 auf das Haar

(b) Erhitzen des Haares zum wirksamen Aushärten.

11. Verfahren nach Anspruch 10, dadurch gekennzeichnet, daß das Haar alkaligeschädigt ist.

12. Verfahren nach Anspruch 10 oder 11, dadurch gekennzeichnet, daß das Haar unbehandelt ist.

13. Verfahren nach allen Ansprüchen 10 bis 12, dadurch gekennzeichnet, daß mit dem ersten Schritt (a) das Haar in die gewünschte Konfiguration gebracht wird.

14. Verfahren nach allen Ansprüchen 10 bis 13, dadurch gekennzeichnet, daß das Haar mit Alkali oder Thioglycolat vorbehandelt ist, bevor die Zusammensetzung aufgetragen wird.

**Revendications**

1. Composition pour la renforcement de la résistance à la traction des cheveux, comprenant:

(a) un alkyltrialcoxysilane de formule:

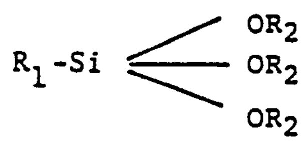

dans laquelle:

$R_1$ est un radical aliphatique ayant 1 à 18 atomes de carbone; un radical aliphatique substitué choisi parmi amino-3 propyle, N,N-diéthyl-amino-3-propyle, N-(propyl)urée ou un alcényle;

$R_2$ est un radical aliphatique ayant 1 à 4 atomes de carbone; et

(b) un solvant dudit alkyltrialcoxysilane.

2. Composition selon la revendication 1, dans laquelle le radical aliphatique est un radical méthyle, éthyle, propyle, butyle, hexyle, octyle, décyle ou octadécyle.

3. Composition selon la revendication 1, dans laquelle l'alcényle est vinyle ou allyle.

4. Composition selon l'une des revendications 1 à 3, dans laquelle le solvant est l'eau, l'éthanol, l'alcool isopropylique ou le nonoxynol-4.

5. Composition selon l'une des revendications 1 à 3, dans laquelle le solvant est une solution aqueuse contenant de l'éthanol, de l'alcool isopropylique ou du nonoxynol-4.

6. Composition selon la revendication 5, dans laquelle l'eau ne représente pas plus d'environ 20% p/p du solvant.

7. Composition selon l'une des revendications 1 à 6, dans laquelle l'alkyltrialcoxysilane est présent à raison d'environ 0,5 à 40% p/p sur la base du volume total de la composition.

8. Composition selon l'une des revendications 1 à 7, dans laquelle, sur la base du volume total de la composition, l'alkyltrialcoxysilane est présent en une quantité allant d'environ 2 à 10% p/p dans 98—90% p/p d'un solvant.

9. Composition selon l'une des revendications 1 à 8, dans laquelle l'alkyltrialcoxysilane est l'octadécyltriéthoxysilane, le n-octyltriéthoxysilane, l'amino-3 propyltriéthoxysilane, le vinyltriéthoxysilane, la n-(triethoxysilylpropyl)urée ou l'éthyltriéthoxysilane.

10. Procédé pour le renforcement des cheveux, comprenant les étapes consistant à:

(a) appliquer sur les cheveux une quantité efficace de la composition telle que définie à l'une des revendications 1 à 9;

(b) appliquer de la chaleur aux cheveux pour effectuer un durcissement.

11. Procédé selon la revendication 10, dans lequel les cheveux sont des cheveux affaiblis par un alcali.

12. Procédé selon l'une des revendications 10 ou 11, dans lequel les cheveux sont des cheveux vierges.

13. Procédé selon l'une des revendications 10 à 12, dans lequel, avant l'étape (a), les cheveux sont placés dans la configuration désirée.

14. Procédé selon l'une des revendications 10 à 13, dans lequel les cheveux sont soumis à un pré-traitement par un alcali ou un thioglycolate avant l'application de la composition.